# EUROPEAN PATENT SPECIFICATION

(11) **EP 1 212 033 B1**
(45) Date of publication and mention of the grant of the patent: **14.10.2009**
(21) Application number: 00961251.6
(22) Date of filing: 29.08.2000
(51) Int. Cl.: A61Q 5/00, A61Q 9/02, A61Q 15/00, A61Q 19/00, A61K 8/04, A61K 8/31

(54) **AQUEOUS LOW PRESSURE COSMETIC COMPOSITION COMPRISING A PROPELLANT, A THICKENER AND A SURFACTANT**
WÄSSRIGE KOSMETISCHE ZUBEREITUNG BEI NIEDRIGEM DRUCK TREIBMITTEL, VERDICKUNGSMITTEL UND TENSID ENTHALTEND
COMPOSITION COSMETIQUE

(30) Priority: 30.08.1999 DE 19941933; 06.01.2000 EP 00200043
(43) Date of publication of application: 12.06.2002
(73) Proprietor: Sara Lee/DE N.V., 3532 AA Utrecht (NL)
(72) Inventor: VOSS, Eckart, Karl, Heinz, NL-2566 KA The Hague (NL); KNEBEL, Silke, Katharina, 40239 Düsseldorf (DE); MONREAL, Michele, 51515 Kurten (DE); HENSEN, Herman, 42781 Haan (DE); SCHMIEDEL, Peter, 40599 Düsseldorf (DE); WITHELL, Trevor, Keith, 40489 Düsseldorf (DE); NIEMAN, Gerrit, NL-2728 AE Zoetermeer (NL)
(74) Representative: Prins, Adrianus Willem
(86) International application number: PCT/NL2000/000599
(87) International publication number: WO 2001/015659

(56) References cited:
- EP-A1- 0 468 555
- EP-A2- 0 919 219
- WO-A1-00/39273
- WO-A1-00/39273
- WO-A1-97/20626
- WO-A1-99/38490
- DE-A- 3 839 349
- DE-A1- 3 839 349
- DE-A1- 4 315 405
- GB-A- 1 121 563
- US-A- 3 541 581
- US-A- 4 753 747
- US-A- 4 981 677
- US-A- 5 679 324
- US-A- 5 879 669
- US-A- 5 902 225

## Description

The invention relates to a cosmetics composition and to its use.

Cosmetics compositions are nowadays available in many different forms. One product having a very distinct appearance from another product may nevertheless serve the same purpose as said other product. Examples of the wide variety that cosmetics may have include lotions, gels, creams, ointments, milks, aerosols, pastes and so forth.

US 5,902,225 and WO 97/20626 describe post-foamable compositions exhibiting multiple-sequential stages of foam. The compositions comprise a foamable utilitarian constituent, a post foaming agent and compressed gas. US 5 879 669 and DE 4315405 describe compositions comprising hydroxyethyl cellulose, ethoxylated castor oil, water, and a propane/butane mixture as a propellant.

US 3,541,581 desribes a post-foamable composition in the form of a stable gel. The gel is to remain substantially free from foaming for at least about 60 seconds. The document uses propellants in an aerosol dispenser, not in the composition.

US 4,735,747 describes a liquid soap neutralization process involving the manufacture of a post foaming gel composition wherein an aqueosu fatty acid mixture is mixed under pressure with a mixture of isopentane and isobutane.

WO 99/38490 relates to an aerosol personal cleansing composition comprising a neat cleansing lotion, a lathering surfactant, a lipophilic skin moisturizing agent, water, and a hydrocarbon propellant. The compositions have either relatively low or relatively high viscosities.

US 5,679,324 pertains to an aerosol foamable fragrance composition which, upon discharging from an aerosol container, forms a fast breaking foam. The composition contains surfactant, a propellant, a fragrance, a thickener, and a cosmetic vehicle wherein the ratio of the surfactant to propellant is from about 1:1 to about 1:10.

WO 00/39273 described a packaged aqueous self-foaming liquid cleansing composition of too viscous a nature to be dispensed from conventional single compartment propellant driven aerosol packaging. A propellant, not part of the composition, is provided in a 'bag in can' type packaging.

DE 38 39 349 discloses post-foaming compositions comprising a soap-free, surfactant-based gel composition consisting essentially of water, a water-soluble anionic alkali metal C10-C16 alkyl ether sulfate surfactant, a water dispersible ethoxylated fatty alcohol or fatty ester, isopropyl myristate, a mono- or disaccharide and a hydrocarbon foaming agent being mixture of n-pentane/isobutane.

The present invention seeks to provide a new form for a cosmetics composition. The objective form is a viscous composition, preferably a gel, which, after application to the skin, creates a post-application foaming effect. When the gel is contacted with the skin, it is desired that a noticeable transition takes place from a gel to a dense creamy foam. To achieve this goal, the invention provides a cosmetics composition comprising a thickener, a propellant, a surfactant and water, wherein the composition is contained in a container under a pressure of no more than 3 bar.

Surprisingly, it has been found that a propellant can be incorporated into a cosmetics composition wherein the resultant pressure is less than that of the propellant on its own. Additionally, the propellant is incorporated into the cosmetics composition substantially without affecting the stability of the composition, even if the composition has the form of a gel. When the composition is applied to the skin, the propellant is released from the gel and the user experiences a foam which is very rich, creamy and long-lasting. It has further been found that the composition is non-flammable and as such is associated with a reduced risk for consumers with respect to fire and explosion hazards.

It is to be noted that cosmetics compositions in the form of a gel and comprising a propellant are known per se. In the field of shaving creams, gels are marketed which, due to the presence of a propellant, convert into a foaming layer or lather when brought into contact with the skin. More recently, this technology has been modified to allow shower foam products to be marketed. These compositions are generally packaged in containers having two compartments, e.g. a bag made of a laminated material suspended inside an aerosol can. Moreover, the amount of propellant needed in these compositions is relatively high. Hence, shaving gels are packed into pressurized containers wherein the pressure usually is as high as 8 bar or more. Consequently, the container needs to be particularly strong and is therefore made of metal.

A composition according to the invention does not require such high amounts of propellant. As has been mentioned, the pressure in a container in which the composition is contained may be as low as 3 bar or less. Preferably, said pressure is lower than 2 bar. Due to this low pressure, the present composition may advantageously be packed in a plastic container, e.g. a polyethylene or polypropylene container, which is economically much more attractive than package in a metal container. Furthermore, a composition according to the invention has been found to have a lower pressure at elevated temperatures than expected. This feature is advantageous in that it can often not be avoided that the product needs to be stored at elevated temperatures for a certain period of time. It is to be noted that the pressure refers to an absolute pressure. Furthermore, the pressure will be in excess of atmospheric pressure, i.e. an overpressure of at least 0.1 bar.

Thus, the invention is a cosmetics composition comprising a thickener, a propellant which is iso-pentane, a surfactant and water, wherein the composition is contained in a container under pressure of at least 0.1 bar in excess of atmospheric pressure and below 3 bar, and wherein the composition has the form of a gel having a viscosity of 10,000 to 50,000 mPas, measured as Brookfield viscosity (23°C, spindel TE, 5 Rpm).

The present composition has the form of a gel. Accordingly, the composition comprises a thickener, which is preferably present in an amount of from 0.01 to 30 wt.%, more preferably from 0.5 to 10 wt.%, even more preferably from 1 to 3 wt.%, based on the weight of the composition. Suitable thickeners are chosen for the compatibility with the propellant and for their capability to provide a stable viscous product, more in particular a stable gel. The viscosity is 10,000 to 50,000, and preferably 20,000 to 30,000 mPas, measured as Brookfield viscosity (23°C, spindle TE, 5 Rpm), e.g. to employ in cleansing gels, face cleansing gels, face care gels, gels for masks, and similar compositions which are commonly used in body care.

Examples of thickeners to be used in compositions according to the present invention include side chain modified polymers based on aerosil-types (hydrophilic silicic acids), polysaccharides such as xanthan gum, guar-guar, agar-agar, alginates and tyloses, carboxymethylcellulose, hydroxyethylcellulose, high molecular polyethylene glycol mono- and diesters of fatty acids, polyacrylates (e.g. Carbopole^{®} of Goodrich or Synthalene^{®} of Sigma), polyacrylamide, polyvinyl alcohols and polyvinylpyrrolidon. Particularly, thickeners with associate action, such as fatty acid glycerides, esters of fatty acids with polyols such as pentaerythrit or trimethylolpropane, fatty alcohol ethoxylates, optionally with EO-homologue distribution, alkyloligoglucosides and sugar esters may be used.

In accordance with the invention, highly preferred thickeners are gums and poly(meth)acrylates, such as polyacrylic acid. Particularly stable gels have been obtained using xanthan gum as the thickener. The presence of a molecular network is highly beneficial to the stability of the gel and can be demonstrated in rheological measurements.

A further important component of the present composition is a propellant. Advantageously, the propellant is chosen for its physico-chemical properties and the character of the foam texture produced on application to skin. A further consideration on which the choice for a suitable propellant may be based is its environmentally friendly character. The propellant is isopentane preferably present in an amount suitable to achieve the desired pressure of the composition in a container in which it is packed. Suitable amounts range from 0.1 to 20 wt.%, preferably from 1 to 15 wt.%, more preferably from 4 to 8 wt.%, based on the weight of the composition.

Furthermore it has been found unexpectedly that alkanes in combination with thickeners, can yield highly viscous mixtures, of a gel-like nature. In cosmetic or pharmaceutical applications, these gel-like mixtures demonstrate a high stability, also when the temperature is decreased. Another advantage of compositions according to the invention may be, the pleasant sensation to the skin, compositions according to the invention may give rise to.

The composition further comprises one or more surfactants. Preferably, the surfactant or surfactants are foaming and skin friendly. Possible surfactants include anionic, nonionic and/or amphoteric surfactants. Typical examples of anionic surfactants include soaps, alkylbenzol sulfonates, alkane sulfonates, olefin sulfonates, alkyl ether sulfonates, glycerine ether sulfonates, α-methylester sulfonates, sulfofatty acids, alkyl sulfates, fatty alcohol ether sulfates, glycerine ether sulfates, fatty acid ether sulfates, hydroxy mixed ether sulfates, monoglyceride (ether) sulfates, fatty acid amide(ether) sulfates, mono- and dialkylsulfosuccinates, mono-and dialkyl sulfosuccinamates, sulfotriglycerides, amide soaps, ether carboxylic acids and salts thereof, fatty acid isothionates, fatty acid sarcosinates, fatty acid taurides, N-acylamino acids such as acylactylate, acyltartrate, acylglutamate and acylaspartate, alkyloligoglucoside sulfates, protein fatty acid condensates and alkyl (ether) phosphates. Typical examples of nonionic surfactants include fatty alcoholpolyglycol ethers , alkylphenolpolyglycol ethers, fatty acid polyglycol esters, fatty acid amidpolyglycol ethers, fatty aminpolyglycol ethers, alkoxylated triglycerides, mixed ethers, optionally partially oxidized alk(en)yloligoglycosides or glucoronic acid derivates, fatty acid-N-alkylclycamides, proteinhydrolysates, polyol fatty acid esters, sugar esters, sorbitan esters, polysorbates and amino-oxides. Typical examples of amphoteric or zwitterionic surfactants include alkyl betains, alkylamido betains, aminopropionates, aminoglycinates, imidazolinium betains and sulfo betains. Further reference, e.g. concerning the preparation of these compounds, may be made to J. Falbe (ed.), "Surfactants in Consumer Products", Springer Verlag. Berlin. 1987, p. 54-124 or J. Falbe (ed.), "Katalysatoren, Tenside und Mineralöladditive", Thieme Verlag, Stuttgart, 1978, p. 123-217. Examples of preferred surfactants include polysorbate 20 or 40, coco glucoside, lauryl glucoside, decyl glucoside, lauryl sulfates such as ammonium, sodium, magnesium, MEA, TEA, or Mipa lauryl sulfate, cocamidopropyl betain, and sodium alkyl sulfosuccinates. The surfactant is preferably present in an amount of from 0.5 to 50 wt.%, more preferably from 2 to 20 wt.% and most preferably from 8 to 13 wt.%, based on the weight of the composition.

It is possible to use a combination of ionic surfactants and amphoteric or non-ionic surfactants. Preferably the ionic surfactant is an anionic surfactant in such combinations. Typically the concentration ranges of such a composition comprising ionic surfactants and amphoteric or non-ionic surfactants are: 0.01 to 30 wt.% of a thickener, 0.1 to 20 wt.% of a hydrophobic compound having an HLB (hydrophilic/lipophilic balance) value of less than 10, 0.5 to 40 wt.% anionic surfactants, 0.25 to 5 wt.% amphoteric surfactants and/or 0.5 to 40 wt.% nonionic surfactants. Furthermore such combinations are preferably used in a ionic surfactant to amphoteric/non-ionic surfactant of in the range of 2:1 to 8:1 wt. to wt, more preferably in the range of 4:1 to 6:1 wt. to wt..

The present compositions may further comprise fatty alcohols, by which primary aliphatic alcohols of the formula R₁OH are meant, in which R₁ is an aliphatic hydrocarbon group containing 6 to 22, preferably 10 to 18 carbon atoms and 0, 1, 2 or 3 double bonds. Typical examples are capron alcohol, capryl alcohol, 2-ethylhexyl alcohol, caprin alcohol, lauryl alcohol, isotridecyl alcohol, myristyl alcohol, cetyl alcohol, palmoleyl alcohol, stearyl alcohol, isostearyl alcohol, oleyl alcohol, elaidyl alcohol, petroselinyl alcohol, linolyl alcohol, linolenyl alcohol, elaeostearyl alcohol, arachyl alcohol, gadoleyl alcohol, behenyl alcohol, erucyl alcohol, and brassidyl alcohol and mixtures thereof. These compounds may be present in amounts of 0.1 to 20, preferably 0.5 to 10 wt.% based on the weight of the composition.

The present compositions may further comprise fatty alcohol ethoxylates, which may have the formula R₂O(AlkO)ₘH, in which R₂ is an aliphatic hydrocarbon group containing 6 to 22, preferably 10 to 18 carbon atoms and 0, 1, 2 or 3 double bonds, m is an integer from 1 to 30, preferably 5 to 20, more preferably 10 to 15, and AlkO is an alkylene oxide. AlkO may be chosen from ethylene oxide, propylene oxide and/or butylene oxide. These compounds may be present in amounts of 0.1 to 20, preferably 0.5 to 10 wt.% based on the weight of the composition.

Depending on the envisaged purpose of the composition, one or more other ingredients may be present. Examples of such ingredients include pH regulating agents, oil bodies, emulsifying agents, preservatives, perfumes, moisturizing agents, UV-filters, emollients, superfatting agents, brighteners, strength improving agents, silicon agents, fats, waxes, lecithins, phospholipids, stabilizing agents, anti-bacterial agents and other bioactive agents, odor-absorbing agents, antiperspirants, antidandruff agents, film-forming agents, swelling agents, antioxidants, insect-repellents, hydrotropes, tanning agents, tyrosin inhibitors, solubilizers and colorants. The composition may further comprise a conventional cosmetics base, such as water, oil, ointment etc..

The composition is preferably formulated to be a gel. In a preferred embodiment the gel transforms upon dispensing from a container into a soft and foamy mousse which cleans in a soft and silky manner.

The pH of the composition is preferably regulated to be close to the pH of the skin itself. Accordingly, the pH of the composition is preferably slightly acidic to slightly alkaline, e.g. in the range of 5 to 8. An example of a suitable pH regulating agent is citric acid. The skilled person will be aware of numerous suitable pH regulating agents that may be employed in the present type of compositions. The amount of the pH regulating agent present is of course adjusted so that the desired pH is achieved.

Examples of suitable oil bodies are guarbeta-alcohols based upon fatty alcohols containing 6 to18 or preferably 8 to 10 carbon atoms, esters of linear C₆-C₂₂ fatty acids with linear C₆-C₂₂ fatty alcohols, esters of branched C₆-C₁₃ Carboxylic acids with linear C₆-C₂₂ fatty alcohols, such as myristyl-myristate, myristyl-palmitate, myristyl-stearate, myristyl-isostearate, myristyl-oleate, myristyl-behenate, myristyl-erucate, cetyl-myristate, cetyl-palmitate, cetyl-stearate, cetyl-isostearate, cetyl-oleate, cetyl-behenate, cetyl-erucate, stearyl-myristate, stearyl-palmitate, stearyl-stearate, stearyl-isostearate, stearyl-oleate, stearyl-behenate, stearyl-erucate, isostearyl-myristate, isostearyl-palmitate, isostearyl-stearate, isostearyl-isostearate, isostearyl-oleate, isostearyl-behenate, isostearyl-oleate, oleyl-myristate, oleyl-palmitate, oleyl-stearate, oleyl-isostearate, oleyl-oleate, oleyl-behenate, oleyl-erucate, behenyl-myristate, behenyl-palmitate, behenyl-stearate, behenyl-isostearate, behenyl-oleate, behenyl-behenate, behenyl-erucate, erucyl-myristate, erucyl-palmitate, erucyl-stearate, erucyl-isostearate, erucyl-oleate, erucyl-behenate and erucyl-erucate. Other examples are esters of linear C₆-C₂₂ fatty acids with branched alcohols, in particular with 2-ethylhexanol, esters of carboxylic acids with linear or branched C₆-C₂₂ fatty alcohols, in particular dioctylmalates, esters of linear and/or branched fatty acids with multivalent alcohols, such as propyleneglycol, dimerdiols or trimertriols, and/or guarbeta-alcohols, triglycerides based upon C₆-C₁₀ fatty acids, liquid mono-/di-/triglyceride mixtures based upon C₆-C₁₈ fatty acids, esters of C₆-C₂₂ fatty alcohols and/or guarbeta-alcohols with aromatic carboxylic acids, in particular benzoic acid, esters of C₂-C₁₂ dicarboxylic acids with linear or branched alcohols having 1 to 22 carbon atoms or polyols with 2 to 10 carbon atoms and 2 to 6 hydroxyl groups, oils of vegetable origin, branched primary alcohols, substituted cyclohexanes, linear and branched C₆-C₂₂ fatty alcohol carbonates, guarbeta-carbonates, esters of benzoic acid and linear and/or branched C₆-C₂₂ alcohols, such as Finsolv® TN, linear or branched, symmetric or asymmetric dialkylethers having 6 to 22 carbon atoms per alkyl group, ring opening products of epoxy fatty acid esters and polyols, silicon-oils and/or aliphatic respectively naphtalenic hydrocarbons, such as squalane, squalene or dialkylcyclohexanes.

An emollient or moisturizing agent may be present in order to improve the ease of application of the composition and the final skin feel the user experiences. Examples of suitable emollients or moisturizing agents include glycerin, propenylglycol, PEG 7 glyceryl cocoate, PEG 6 caprylic or capric glycerides, glyceryl oleate and lipids in general, such as paraffin oil or polar oils. An emollient or moisturizing agent is preferably present in an amount ranging from 0.5 to 15 wt.%, based on the weight of the composition.

Suitable emulsifying agents are for example non-ionic surfactants such as:
- reaction products of 2 to 30 mole ethylene oxide and/or 0 to 5 mole propylene oxide with linear fatty alcohols having 8 to 22 carbon atoms, with fatty acids having 12 to 22 carbon atoms, with alkylphenols having 8 to 15 carbon atoms in the alkyl group and also with alkylamines having 8 to 22 carbon atoms in the alkyl group;
- alkyl and/or alkenyl-oligoglycosides having 8 to 22 carbon atoms in alk(en)yl group and ethoxylated analogs thereof;
- reaction products of 1 to 15 mole ethylene oxide and castor oil and/or fixated castor oil;
- reaction products of 15 to 60 mole ethylene oxide and castor oil and/or fixated ricinoleic oil;
- partial-esters of glycerin and/or sorbitane with unsaturated, linear or saturated, branched fatty acids having 12 to 22 carbon atoms and/or hydroxycarboxylic acids having 3 to 18 carbon atoms, as well as adducts thereof with 1 to 30 mole ethylene oxide;
- partial-esters of polyglycerin (average self-condensation degree 2 to 8), polyethyleneglycol (molecular weight of 400 to 5000), trimethylolpropane, pentaerythrite, sugar-alcohols, such as sorbitol, alkylglucosides, such as methylglucoside, butylglucoside, laurylglucoside, as well as polyglucosides, such as cellulose, with saturated and/or unsaturated, linear or branched fatty acids having 12 to 22 carbon atoms and/or hydroxycarboxylic acids having 3 to 18 carbon atoms, as well as adducts thereof with 1 to 30 mole ethylene oxide;
- mixed-esters of pentaerythrite, fatty acids, citric acid and fatty alcohols in accordance with DE 1165574 PS and/or mixed-esters of fatty acids having 6 to 22 carbon atoms, methylglucose and polyols, preferably glycerin or polyglycerin;
- mono-, di- and trialkylphosphates, as well as mono-, di- and/or tri-PEG-alkylphosphates and salts thereof;
- wool-wax alcohols;
- polysiloxan-polyalkyl-polyether-copolymers respectively derivatives thereof;
- polyalkelyneglycols;
- glycerincarbonates;

The reaction products of ethylene oxide and/or propylene oxide with fatty alcohols, fatty acids, alkylphenols or ricinolic oils are commercially obtainable products. They are available as homologous mixtures, of which the average alkoxylation degree is in accordance with the mass ratio of ethylene oxide and/or propylene oxide and substrate, with which the reaction takes place. C₁₂-C₁₈ fatty acid mono- and diesters of reaction products of ethylene oxide and glycerin are known in relation to cosmetic compositions from DE-2024051.

Typical examples of suitable partial-glycerides are monoglyceride-hydroxy-stearinate, diglyceridehydroxy-stearinate, monoglyceride-isostearinate, diglyceride-isostearinate, monoglyceride-oleiate, diglyceride-oleiate, monoglyceride-ricinoleate, diglyceride-ricinoleate, monoglyceride-linoleate, diglyceride-linoleate, monoglyceride-linolenate, diglyceride-linolenate monoglyceride-erucate, diglyceride-erucate, monoglyceride-tartrate, diglyceride-tartrate, monoglyceride-citrate, diglyceride-citrate, monoglyceride-malate, diglyceridemalate, as well as technical mixtures thereof, which may still contain small amounts of triglyceride, depending upon the production process. Reaction products of 1 to 30, preferably 5 to 10 mole ethylene oxide with aforementioned partial-glycerides are suitable too.

Examples of suitable sorbitol-esters are sorbitol-monoisostearate, sorbitol-sesquiisostearate, sorbitol-diisostearate, sorbitol-triisostearate, sorbitol-monooleate, sorbitol-sesguioleate, sorbitol-dioleate, sorbitol-trioleate, sorbitol-monoerucate, sorbitol-sesguierucate, sorbitol-dierucate, sorbitol-tri-erucate, sorbitol-monoricinoleate, sorbitol-sesquiricinoleate, sorbitol-di-ricinoleate, sorbitol-triricinoleate, sorbitol-monohydroxystearate, sorbitol-sesquihydroxy-stearate, sorbitol-dihydroxystearate, sorbitol-trihydroxystearate, sorbitol-monotartrate, sorbitol-sesquitartrate, sorbitol-ditartrate, sorbitol-tritartrate, sorbitol-monocitrate, sorbitol-sesquicitrate, sorbitol-di-citrate, sorbitol-tricitrate, sorbitol-monomaleate, sorbitol-sesquimaleate, sorbitol-dimaleate, sorbitol-trimaleate as well as technical mixtures thereof. Reaction products of 1 to 30, preferably 5 to 10 mole ethylene oxide with said sorbitol esters are suitable too.

Typical examples of suitable polyglycerin esters are polyglyceryl-2 dipolyhydroxy-stearate (Dehymuls® PGPH), polyglycerin-3-diisostearate (Lameform® TGI), polyglyceryl-4 isostearate (Isolan® G1 34), polyglyceryl-3 oleate, diisostearoyl polyglyceryl-3 diisostearate (Isolan® PDI), polyglyceryl-3 methylglucose distearate (Tego Care® 450), polyglyceryl-3 beeswax (Cera Bellina®), polyglyceryl-4 caprate (Polyglycerol Caprate T20 10/90), polyglyceryl-3 cetyl ether (Chimexane® NL), polyglyceryl-3 distearate (Cremophor® GS 32) and polyglyceryl polyricin-oleate (Admul® WOL 1403) polyglyceryl dimerateisostearate, as well as, mixtures thereof.

Further examples of suitable polyol esters are possibly with 1 to 30 mole ethylene oxide derivatized mono-, di- and triesters of trimethylolpropane or pentaerythrite with lauric acid, cocinic acid, palmic acid, talcum-oil acid, palmic acid, stearic acid, oleic acid, behenic acid and the like.

Cationic surfactants may also be suitable emulsifying agents. Preferred cationic surfactant are quaternary esters, in particular quaternary methyl-di-fatty acid- triethanol-amine-ester salts.

Superfatting agents may be compounds such as lanolin or lecithin as well as polyethoxyated or acylated lanolin- and lecithin derivatives, polyol fatty acid esters, monoglycerides and fatty acid alkanolamides, wherein fatty acid alkanolamides also tend to stabilizing the foam.

Brighteners may for example be selected from: alkylene glycolesters, in particular ethylene glycoldistearate, fatty acid alkanolamide, in particular cocinic acid diethanolamide; partial-glycerides, especially monoglyceridestearate; esters of multivalent, possibly hydroxyl substituted carboxylic acids with fatty alcohols having 6 to 22 carbon atoms, in particular esters with long chains of tartaric acid; fatty compounds, such as fatty acids, fatty ketones, fatty aldehydes, fatty ethers and fatty carbonates, which have a total of at-least 24 carbon atoms, in particular laurin and distearyl ether; fatty acids such as stearic acids, hydroxystearic acids or behenic acids, ring opening products of olefinepoxides having 12 to 22 carbon atoms with fatty alcohols having 12 to 22 carbon atoms and/or polyols having 2 to 15 carbon atoms and 2 to 10 hydroxyl groups, as well as mixtures thereof.

As strength improving agents, particularly suitable groups of compounds are e.g. C₁₂-C₂₂ fatty alcohols or hydroxy- fatty alcohols, preferably having 16 to 18 carbon atoms. Partial-glycerides, fatty acids or hydroxy fatty acids are also examples of suitable strength improving agents. Preferred is a combination of these compounds with alkyloligoglucosides and/or fatty acids-N-methylglucamides of equal chain length and/or polyglycerinpoly-12-hydroxystearates.

Examples of suitable silicon compounds are dimethylpolysiloxanes, methylphenylpolysiloxanes, cyclic silicons, as well as amino-, fatty acid-, alcohol-, epoxy-, fluoro-, glycoside-, and/or alkylated silicon compounds, which may either be in a liquid phase or a resin phase, at room temperature. Other examples are dimethicones, in particular mixtures of dimethicones having an average chain length of 200 to 300 dimethylsiloxane moieties and hydrated silicates. A detailed overview of suitable volatile silicons can be found in Todd et al., Cosm. Toil. 91, 27 (1976).

Typical examples of fats are tri-glycerides. Suitable waxes are for example natural waxes, such as candililla wax, carnauba wax, Japan wax, esparto grass wax, ceric wax, guaruma wax, rice bran wax, sugar cane wax, orycury wax, montan wax, bees wax, shellac wax, walrat, lanolin (wool wax), tail root fat, ceresin, ozocerite (earth wax), petrolatum, paraffin wax, micro waxes, chemically modified waxes (hard waxes) such as montan ester waxes, sasol waxes, hydrated jujube waxes, as well as synthetic waxes, such as polyalkylene waxes and polyethyleneglycol waxes. In addition to fats, certain compounds that are similar to fats may be added, such as lecithins and phospholipids. With lecithins, the person skilled in the art means those glycero-phospholipids, which can be formed by esterification of fatty acids, glycerin, phosphoric acids and choline. In the art, lecithins are therefor also often referred to as phospatidylcholine (PC) and can be characterized by the following general formula: 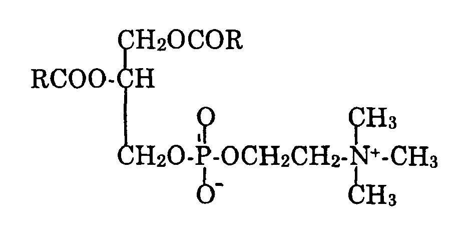 wherein R typically represents linear aliphatic hydrocarbon moieties having 15 to 17 carbon atoms and up to 4 cis-double bonds. Examples of natural lecithins are compounds from the group of Cephalins, which are also referred to as phosphatide acids, and derivatives of 1,2-diacyl-sn-glycerin-3-phosphoric acids.

Examples of suitable phospholipids are mono-esters and, preferably, di-esters of phosphoric acids and glycerin (i.e. glycerin phosphates), which are generally regarded as fatty substances. Sphingosines or better sphingolipids are other examples of suitable additives.

Metal salts of fatty acids, such as magnesium- aluminum- and/or zinc stearate respectively -ricinoleate can be employed as stabilizing agents.

Suitable biologically active additives include tocopherol, tocopherol acetate, tocopherol palmitate, ascorbic acid, desoxyribonucleinic acid, retinol, bisabolol, allantoin, phytantriol, panthenol, AHA acids, amino acids, ceramides, pseudoceramides, essential oils, plant extracts and vitamin C complexes.

Cosmetic anti bacterial agents are active against the development of body odors. Body odors develop due to the activity of dermal bacteria on apocryne perspiration, during which unpleasantly smelling metabolites are formed.

Suitable anti bacterial agents include germination inhibiting compounds which are in principle active against all gram positive bacteria, such as 4-hydroxybenzoic acid and salts plus esters thereof, N-(4-chlorophenyl)-N'-(3,4 dichlorophenyl)urea, 2,4,4'-trichloro-2'-hydroxy-diphenyl ether (Triclosan), 4-chloro-3,5-dimethylphenol, 2,2'-methylene-bis(6-brom-4-chlorophenol), 3-methyl-4-(1-methylethyl)phenol, 2-benzyl-4-chlorophenol, 3-(4-chlorophenoxy)-1,2-propandiol, 3-iodo-propinylbutyl carbamate chlorohexidin, 3,4,4'-trichloro carbanilide (TTC), antibacterial fragrant compounds, thymol, thymian oil, eugenol, clove oil, menthol, mint oil, fernesol, phenoxyethanol, glycerin-monolaurate (GML), diglycerin-monocaprinate (DMC), salicylic acid-N-alkylamide such as salicylic acid-n-octylamide or salicylic acid-n-decylamide. Furthermore enzyme inhibitors can be used to help to prevent the production of undesired body odors. Esterase inhibitors, for example, are suitable for this purpose in compositions according to the invention. Preferred enzyme inhibiting agents are trialkylcitrates, such as trimethylcitrate, tripropylcitrate, triisopropylcitrate, tributylcitrate and in particular triethylcitrate (Hydragen® Cat, Henkel KGaA, Dusseldorf/FRG). The compounds inhibit the enzyme activity and reduce the formation of odorous compounds. Other suitable esterase inhibiting compounds are for example sterolsulphates or -phosphates, such as lanosterin-, cholesterin-, campesterin-, stigmasterin and sitosterinsulphate respectively -phosphate. Dicarboxylic acids and esters thereof, such as glutaric acids, glutaric acid mono-ethylesters, adipinic acid, adipinic acid monoethylester, adipinic acid diethylester, malonic acids and malonic acid diethyl ester, hydroxycarboxylic acids and esters thereof such as citric acid, malic acid, tartaric acid or tartaric acid diethylester as well as zinc glycinate.

In addition odor-absorbing agents may be used to suppress the formation of a undesired scent. Suitable compounds decrease the partial pressure of the single components and as such decrease the velocity of spreading. It is important that perfume compositions are not absorbed significantly. Odor-absorbing agents are normally not directly active against bacteria. They comprise for example as primary component a complex of a zinc salt of ricinoleic acid or special fragrance-neutral perfume compounds, known to the skilled professional as fixatives. Examples of these fixatives are labdanum extracts respectively Styrax or certain abietic acid derivatives. Furthermore perfume compounds, including fragrant oils, may serve as masking agents and also may give a typical fragrant character to compositions. Examples of fragrant oils are mixtures of natural and synthetic fragrant compounds. Examples of natural fragrant compounds are extract of flowers, stems, leafs, fruits, fruit skin, fruit peel, rots, woods, herbs, grasses, needles and branches, as well as resins and balms. Furthermore materials of animal origin are suitable, such as civet or castoreum. Typical synthetic fragrant compounds are esters, ethers, aldehydes, ketones, alcohols and hydrocarbons. Examples of fragrant esters as benzylacetate, p-tert-butylcyclohexylacetate linalyl acetate, phenylethylacetate, benzylbenzoate, benzylformiate, allylcyclohexyl-proprionate, styrallylproprionate and benzylsalicytate. An example of a suitable ether is benzylethyl ether. Examples of suitable aldehydes are linear alkanals having 8-18 carbon atoms, citral, citronellal, citronellyloxyacetyl aldehyde, cyclamen aldehyde, hydroxycitronellal, lillial and bourgeonal. Examples suitable ketones are jonones and methylcedrylketone. Examples of suitable alcohols are anethol, citronellol, eugenol, isoeugenol, geraniol, linalool, phenylethyl alcohol and terpineol and examples, of the hydrocarbons mainly terpenes and balms. Preferably mixtures of different fragrant compounds, resulting in a pleasant aroma, are employed. Also commonly used aromatic compounds of the group of etheric oils of low volatility, are suitable perfume oils. Examples of these are sage oil, camille oil, clove oil, balm mint oil, mint oil, cinnamon oil, linden-blossom oil, juniper oil, vetiver oil, olibanum oil, galbanum oil, labdanum oil and lavender oil. Preferred are bergamot oil, dihydromyrcenol, lilial, lyral, citronellol, phenylethyl alcohol, α-hexylcinnamon aldhyde, geraniol, benzylacetone, cyclamen aldehyde, linalool, boisambrene forte, ambroxan, indol, hedione, sandelice, citron-oil, mandarin-oil, orange-oil, allylamylglycolate, cyclovertal, lavender-oil, muscate sage-oil, β-damascone, geranium-oil bourbon, cyclohexylsalicylate, vertofix coeur, iso-E-super, fixolide NP, evemyl, iraldein gamma, phenyl-acetic acid, geranyl acetate, benzyl acetate, rose-oxide, romilate, irotyl and floramate, either employed alone or in a mixture.

Anti-perspirants reduce the formation of perspiration by influencing the activity of exocryne perspiratory glands, and as such help to prevent wetting of arm pits as well as the formation of body odors. Aqueous or non aqueous compositions of Anti-perspirants typically comprise the following ingredients:
- astringent agents;
- oil compounds;
- non ionic emulsifying agents;
- co-emulsifying agents;
- strength improving agents;
- aiding compounds such as thickening agents or complexing agents; and/or
- non-aqueous solvents such as ethanol, propyleneglycol; and/or glycerin.

Examples of astringent agents are in particular all salts of aluminum, zirconium and zinc, such as aluminumchloride, aluminumchlorohydrate, aluminumdichlorohydrate, aluminumsesqui-chlorohydrate, and complexes thereof, e.g. with propyleneglycol-1,2. Aluminumhydroxyallantoinate, aluninumchloridetartrate, aluminum-zirconium-trichlorohydrate, aluminum-zirconium-tetrachlorohydrate, aluminum-zirconium-pentachlorohydrate and complexes thereof, for example with amino acids such as glycin. In addition anti-perspirants may comprise the usual oil soluble and water soluble aiding agents in lower concentrations. Examples of oil soluble aiding agents are:
- infection inhibiting, skin protecting or fragrant etheric oils;
- synthetic skin protecting agents; and/or
- oil soluble perfume oils.

Suitable antidandruff agents are for example Octopirox® (1-hydroxy-4-methyl-6-(2,4,4-trimethylpentyl)-2-(1H)-pyridon-monoethanolamine salt, Babypival, Pirocton Olamin, Ketoconazol®, (4-acetyl-1-{-4-[2-(2,4-dichlorophenyl)r-2-(1H-imidazol-1-ylmethyl)-1,3-dioxylane-c-4-ylmethoxyphenyl} piperazin, selenedisulfide, sulfur colloidals, sulfurpolyethyleneglycolsorbitol-monooleate, sulfurricin-polyethoxylate, sulfurtar destillate, salicylic acid (in particular in combination with hexachlorophen), undexylene acid monoethanolamide sulfosuccinate Na-salt, Lamepon® UD (protein-undecylene acid condensate, zincpyrethione, aluminumpyrethione and magnesiumpyrethione/dipyrethione-magnesiumsulfate.

Usual film-forming agents include chitosan, micro crystalline chitosan, quaternary chitosan, polyvinylpyrolidon, vinylpyrolidon-vinylacetate-copolymerisate, polymers of acrylic acid, quaternary cellulose derivatives, collagen, hyaluronic acid, respectively salts thereof and similar compounds.

Suitable swelling agents for aqueous phases include montmorillonites, clay mineral compounds, pemules, as well as alkylated carbopoltypes (Goodrich). Furthermore, polymers suitable as swelling agents can be found in the overview by R. Lochhead in cosm. Toil. 108, 95 (1993).

Suitable UV-filters are for example compounds - liquid or crystalline at room temperature - that are capable of absorbing ultraviolet radiation and of releasing the absorbed energy in the form of electromagnetic radiation of a longer wavelength, e.g. in the form of infra red radiation. UVB filters may be oil soluble or water soluble. Examples of oil soluble compounds are:
- 3-benzylidencamphor respectively 3-benzylidennorcamphor and derivatives thereof, such as 3-(4-mathylbenzyliden)camphor as further described in EP 0693471;
- 4-aminobenzonic acid derivatives, preferably 4-dimethylaminobenzoic acid-2-ethylhexyl ester, 4-(dimethylamino)benzoic acid-2-octyl ester and 4-dimethylamino)benzoic acid amyl ester;
- esters of cinnamon acid, preferably 4-methoxy-cinnamon-2-ethylhexyl ester, 4-methoxy-cinnamon acid propyl ester, 4-methoxy-cinnamon acid isoamyl ester 2-cyano-3,3-phenyl cinnamon acid-2-ethylhexyl ester (octocrylene);
- esters of salicylic acid, preferably salicylic acid-2-ethylhexyl ester, salicylic-4-isopropylbenzyl ester, salicylic acid homomenthyl ester;
- derivatives of benzophenones, preferably 2-hydroxy-4-methoxybenzophenone, 2-hydroxy-4-methoxy-4'-methylbenzophenone, 2,2'di-hydroxy-4-methoxybenzophenone;
- esters of benzalmalon acid, preferably 4-methoxybenzamalon acid di-2-ethylhexyl ester;
- triazin derivatives, e.g. 2,4,6-trianilino-(p-carbo-2'-ethyl-1'-hexyloxy)-1,3,5-triazin and octyl triazon, as described in EP 0 818 450 A1 or dioctyl butamido triazone (Uvasorb® HEB);
- propane-1,3-dione, e.g. 1-(4-tert butylphenyl)-3-(4'methoxyphenyl)propane-1,3-dione;
- ketotricyclo(5.2.1.0)decane-derivatives as described in EP0694521B1.

Examples of water soluble UV-filters are:
- 2-phenylbenzimidazol-5-sulfonic acid and alkali-, earth alkali-, ammonium-, alkylammonium-, alkanolammonium- and glucammonium salts thereof;
- sulfonic acid derivatives of benzophenon, preferably 2-hydroxy-4-methoxybenzophenone-5-sulfonic acid and salts thereof;
- sulfonic acid derivatives of 3-benzylidencamphors, e.g. 4-(2-oxo-3-bornylidenemethyl)benzol-sulfonic acid and 2-methyl-5-(2 oxo-3-bornylidene)sulfonic acid and salts thereof.

Typical examples of UV-A filters are derivatives of benzoylmethane, such as 1-(4'-tert.butylphenyl)-3-(4'-methoxyphenyl)-propane-1,3-dione as well as enamine compounds as described in DE 19712033 A1 (BASF). Naturally it is possible to employ mixtures of UV-A and UV-B filters. In addition to the already mentioned soluble compounds, non-soluble sun-screen pigments may also be employed. In particular small dispersed metal oxide particles and metal salts, such as zinc oxide, titanium dioxide, oxides of respectively iron, zirconium, silicium, manganese, aluminum and cerium, as well as mixtures thereof, the salts of silicates (talcum), barium sulfate and zinc stearate. The oxides and salts are employed in compositions for skin care or skin protective emulsions and in decorative cosmetics. The particles should have an average diameter of less than 100 nm, preferably between 5 and 50 nm, more preferably between 15 and 30 nm. The particles may have a spherical, ellipsoidal or other shape. Optionally the surfaces of pigments may have been treated, i.e. by hydrophilization or hydrophobization. Typical examples are coated titanium dioxide, such as titanium dioxide T 805 (Degussa) or Eusolex® T2000 (Merck). Typical examples of hydrophobic coating agents are silicones and particularly trialkoxyoctylsilanes or Simethicones. So called micro- or nanopigments are preferably employed in sun screen compositions. Preferably micronized zinc oxide is used. Further examples of suitable UV-filters can be found in the overview of P. Finkel in SÖFW-Journal 122, 543 (1996).

In addition to the groups of primary light protective agents, as mentioned above, it is also possible to use secondary light protective agents of the group of antioxidants which can stop photochemical reaction chains. These photochemical reactions are induced by UV-radiation as it enters the skin. Typical examples of suitable antioxidants are amino acids such as glycine, histidin, tyrosine, tryptophane, and derivatives thereof, imidazols such as urocaninic acid, and derivatives thereof, peptides, e.g. D,L-carnosin, D-carnosin, L-carnosin and their derivatives (e.g. anserin), carotinoids, carotins, (e.g. α-carotin, β-carotin, lycopin) and derivatives thereof, chlorogenic acid and their derivatives, liponic acids and their derivatives (e.g. dihydroliponic acid), aurothioglucose, propylthiouracil, and other thiols (e.g. thioredoxin, glutathion, cystein, cystin, cystamin,and glycosyl-, N-acetyl-, methyl-, ethyl-, propyl-, amyl-, butyl- and lauryl-, palmitoyl-, oleyl-, γ-linoleyl-, cholesteryl- and glyceryl-ester), as well as, salts thereof, dilaurylthiodipropionate distearylthiodipropionate thiodipropionic acid and derivatives thereof (esters, ether, peptide, lipide, nucleotide, nucleoside and salts), as well as, sulfoximins (e.g.buthioninisulfoximine, homocysteinsulfoximine, butioninsulfone-, penta-, hexa-, heptathioninsulfoximine) in very low tolerable concentrations (e.g. pmol to µmol/kg), further (metal)-chelators (e.g. α-hydroxy fatty acids, palmitine acids, phytine acids, lactoferrin), α-hydroxy acids, such as citric acid, lactic acid or malic acid, humic acid, bile acid, bile extracts, bilirubin, biliverdin, EDTA, EGTA and derivatives thereof, unsaturated fatty acids and derivatives thereof, such as γ-linolenic acid, linoleic acid or oleic acid, foleic acid and derivatives thereof, ubiquinone, ubiquinol, and derivatives thereof, vitamin C and derivatives, such as ascorbylpalmitate, Mg-ascorbylphosphate or ascorbylacetate, tocopherols and derivatives, such as vitamin-E-acetate, vitamin A and derivatives, such as vitamin-A-palmitate, as well as coniferylbenzoate of benzoin resin, rutinic acid and derivatives thereof, α-glycosylrutine, ferulic acid, furfurylidenglucitol, carnosin, butylhydroxytoluol, butylhydroxyanisol, nordihydroguaiaretic acid resin, nordihydroguaiaretic acid, trihydroxybutyrophenon, uric acid and derivatives thereof, mannose and derivatives thereof, superoxide dismutase, zinc and derivatives thereof, such as ZnO and ZnSO₄, selenium and derivatives thereof, such as selenium-methionin, stilbenes and derivatives thereof, such as stilbeneoxide, trans stilbene oxide, and the suitable derivatives (salts, esters, ethers, sugars, nucleotides, nucleosides, peptides, lipids) of the mentioned agents.

In order to improve flowability it is possible to add hydrotropes, such as ethanol, isopropanol, or polyols. Preferred polyols have 2 to 15 carbon atoms and at least 2 hydroxyl groups. The polyols may have additional functional groups, in particular amino groups, respectively they may be modified with nitrogen. Typical examples are:
- glycerin;
- alkyleneglycols, such as ethyleneglycol, diethyleneglycol, propylene glycol, butylene glycol, hexylene glycol as well as polyethyleneglycols with a average molecular weight of 100 to 1000 Dalton;
- technical oligoglycerin mixtures with a self condensation degree of 1.5 to 10 such as technical diglycerin mixtures with a diglycerin concentration of 40 to 50 % w/w;
- methyol compounds,in particular trimethylolethane, trimethylolpropane, trimethylolbutane, pentaerythrite and dipentaerythrite;
- lower alkylglucosides, in particular those having 1 to 8 carbon atoms in alkyl group, such as methyl- and butylglucoside
- sugar alcohols having 5 tot 12 carbon atoms, such as sorbitol or mannitol;
- sugars having 5 to 12 carbon atoms, such as glucose or saccharose
- amino sugars, such as glucamine;
- dialcoholamines such as diethanolamine or 2-amino-1,3-propanediol.

Examples of suitable preservatives are phenoxyethanol, formaldehyde, parabene, pentadiol or sorbic acid, as well as compounds mentioned in "Anlage 6, Teil A und B der Kosmetikverordnung". Examples of suitable insect-repellents are N,N-diethyl-m-toluamide, 1,2-pentadiol or ehtyl butylacetylaminoproprionate. Dihydroxy-aceton is a suitable tanning agent. Suitable tyrosine inhibitors - which prevent the formation of melanin and are employed in depigmentation agents - are for example arbutin, koji acid, coumarinic acid and ascorbic acid (vitamin C).

Suitable perfume oils are mixtures of natural and synthetic fragrant compounds. Natural fragrant compounds include extracts of flowers, such as lily, lavender, rose, jasmine, neroli and yiang-yiang, stems and leafs such as geranium, patchouli, petit-grain, fruits such as aniseed, coriander, cumin, juniper, fruit skins or peels, such as those of bergamot, lemons, oranges, roots such as macis, angelica, celery, cardamom, costus, iris, calmus, woods such as pine, sandal, guaja, cider and rose, herbs and grasses such as, estragon, lemon grass, sage, thyme, needles and branches, grove, spruce, pine, larch, resins and balms, such as galbanum, elimi, benzoe, myrrh, olibanum, opoponax. Furthermore materials of animal origin can be used such as civet and castoreum. Typical synthetic fragrant compounds are esters, ethers, aldehydes, ketones, alcohols and hydrocarbons. Examples of suitable esters are benzylacetate, phenoxyethylisobutyrate, p-tert.-butylcyclohexylacetate, linalylacetate, dimethylbenzylcarbinylacetate, phenylethylacetate, linalylbenzoate, benzylformiate, ethylmethylphenylglycinate, allylcyclohexylpropionate, styrallylproprionate and benzylsalicylate. Examples of suitable ethers are benzylethyl sethers, examples of suitable aldehydes are linear alkanals having 8 to 18 carbon atoms, citral, citronellal, citronellyloxyacetaldehyde, cyclamealdehyde, hydroxycitronellal, lilial and bourgeonal, examples of suitable ketones are jojones, α-isomethylionon and methylcedrylketon, Examples of suitable alcohols are anethol, citronellol, eugenol, iso-eugenol, geraniol, linalool, phenylethylalcohol and terpineol. Primary examples of hydrocarbons are terpenes and balms. Preferably however, mixtures of several fragrant compounds are employed, together resulting in a preferred aroma. In addition, etheric oils of low volatility, are suitable perfume oils. Examples of these are sage oil, camille oil, clove oil, balm mint oil, mint oil, cinnamon oil, linden-blossom oil, juniper oil, vetiver oil, olibanum oil, galbanum oil, labolanum oil and lavender oil. Preferred are bergamot oil, dihydromyrcenol, lilial, lyral, citronellol, phenylethyl alcohol, α-hexylcinnamon aldhyde, geraniol, benzylacetone, cyclamen aldehyde, linalool, boisambrene forte, ambroxan, indol, hedione, sandelice, citron-oil, mandarin-oil, orange-oil, allylamylglycolate, cyclovertal, lavender-oil, muscate sage-oil, β-damascone, geranium-oil bourbon, cyclohexylsalicylate, vertofix coeur, iso-E-super, fixolide NP, evemyl, iraldein gamma, phenyl-acetic acid, geranyl acetate, benzyl acetate, rose-oxide, romilate, irotyl and floramate, either employed alone or in a mixture.

Suitable colorants are any colorants that are suitable for cosmetic purposes, as for example mentioned in the publication "Kosmetische Farbemittel" der Farbstoff-kommission der Deutschen Forschungsgemeinschaft, Verlag Chemie, Weinheim, 1984, S. 81-16". Such colorants are usually employed in concentrations varying from 0.001 to 0.1 % w/w based on the weight of the composition.

The total amount of additives can vary from 1 to 50 preferably from 5 to 40 % w/w based on the weight of the composition.

The balance of the composition will generally be made up by water. Optionally, a small amount of an alcohol, such as ethanol or isopropanol may be present, e.g. to achieve a disinfecting effect. Water will typically be present in an amount ranging from 50 to 95 wt.%, based on the weight of the composition.

The compositions can be prepared according to the usual cool or heated processes; a preferred method of preparation is a phase-inversion temperature method.

Dependent on the chosen ingredients of the composition as set forth above, a cosmetics composition according to the invention may find application as a sun cream or lotion, body milk, shampoo, bathing or shower gel, hair care product, deodorant or moisturizing cream. If desired, the present composition may also be employed in a pharmaceutical setting, for instance as an ointment. In such a case, the composition will further comprise a pharmaceutically active agent or a bioactive agent.

The invention will now be elucidated by the following, non-restrictive examples.

### Example 1

A composition was prepared of the following ingredients in the following amounts (wt.%):

| | | |
|---|---|---|
| - Surfactants: | Magnesium Laureth Sulfate (1) | 11.43 |
| | Lauryl Glucoside (2) | 5.19 |
| - pH regulator: Citric acid | | 0.11 |
| - Preservative: Kathon CG | | 0.06 |
| - Thickener: Xanthan gom | | 0.80 |
| - Moisturizing agent: Glycerin | | 5.00 |
| - Emollient: Cetiol HE | | 2.00 |
| - Conditioning agent: Merquat Plus 3331 | | 1.00 |
| - Perfume | | 1.00 |
| - Coloring agent: Patentblue V E 131 | | 0.0015 |
| - Water | | Balance |

The composition was prepared by first adding the water to a vessel. Next, in subsequent order, the preserative and the thickener were added. These components were mixed and homogenized until the thickener was swollen and fully dispersed. To the obtained dispersion, the surfactants were added separately with mixing to fully disperse the surfactant after each addition. The remaining ingredients, except the citric acid, were then added and mixing was continued until all were fully dispersed. Finally, the pH was adjusted by addition of the citric acid.

This composition was then cooled to below 10°C. The propellant to be added, isopentane, was also cooled to said temperature. The composition and the propellant were mixed with one another while taking care that no air was incorporated at constant temperature. The propellant was added in an amount to finally reach a concentration of 6 wt.%, with respect to the total weight of the final composition. After thorough mixing, the composition was allowed to warm up and brought into a suitable plastic container while still having a temperature below 20°C.

### Example 2

Six compositions were prepared. Three were in accordance with the present invention (A, B, C), and three were not (CA, CB, CC). For the preparation of the compositions, a surfactant mixture comprising a thickener was mixed with pentane at a pH of 5.5-6.5. The viscosities were measured using the Brookfield method at 23°C ('Spindel TE', 5 rpm) in mPas. The results are shown in Table 1.

**Table 1: Gels of surfactant mixtures (amounts in weight percentages)**

| Composition | A | B | C | CA | CB | CC |
|---|---|---|---|---|---|---|
| Texapon^{®} N70 Sodium Laureth Sulfate | 8 | 8 | 4 | 8 | 8 | 4 |
| Plantacare^{®} 818 Coco-Glucosid | 1 | - | 1 | 1 | - | 1 |
| Dehyton^{®} K Cocoamidopropylbetain | 1 | 1 | 1 | 1 | 1 | 1 |
| Glyceryl-PEG-140-tristearate | 4 | 4 | 4 | 4 | 4 | 4 |
| Pentane | 6 | 6 | 6 | - | - | - |
| Water | ad 100 | | | | | |
| Viscosity | | | | | | |
| - 1 hour | 30,500 | 25,000 | 34, 000 | 3000 | 3200 | 2900 |
| - 4 weeks . | 29,600 | 27,000 | 34,500 | 2700 | 2800 | 2600 |

## Claims

1. Cosmetics composition comprising a thickener, a propellant which is iso-pentane, a surfactant and water, wherein the composition is contained in a container under a pressure of at least 0.1 bar in excess of atmospheric pressure and below 3 bar, and wherein the composition has the form of a gel having a viscosity of 10,000 to 50,000 mPas, measured as Brookfield viscosity (23°C, spindle TE, 5 Rpm).

2. Composition according to claim 1, wherein the pressure is no more than 2 bar.

3. Composition according to claim 1 or 2, wherein the container is a plastic container.

4. Composition according to any of the claims 1-3, wherein said composition comprises at least two surfactants and a hydrophobic compound having a HLB-value of less than 10.

5. Composition according to any of the preceding claims, comprising from 0.01 to 30 wt.% of thickener, from 1 to 15 wt.% of propellant, from 0.5 to 50 wt.% of surfactant and the balance being water and other customary body care ingredients.

6. Composition according to any of the preceding claims, wherein the thickener is chosen from the group of gums, poly(meth)acrylates, polymers based upon aerosil-types, polysaccharides, high molecular polyethyleneglycolmono- and diesters of fatty acids, polyacrylamides, polyvinylalcohols, polyvinylpyrrolidons, esters of fatty acids with polyols, fatty alcoholethoxylates, alkyloligoglucosides and sugar-esters.

7. Composition according to claim 6, wherein the thickener is chosen from xanthan gom, guar-guar, agar-agar alginates, tyloses, carboxymethylcellulose, hydroxyethylcellulose.

8. Composition according to any of the preceding claims further comprising one or more ingredients chosen from the group of pH regulating agents, oil bodies, emulsifying agents, preservatives, perfumes, moisturizing agents, UV-fllters, emollients, superfatting agents, brighteners, strength improving agents, silicon agents, fats, waxes, lecithins, phospholipids, stabilizing agents, anti-bacterial agents and other bioactive agents, odor-absorbing agents, antiperspirants, antidandruff agents, film-forming agents, swelling agents, antioxidants, insect-repellents, hydrotropes, tanning agents, tyrosin inhibitors, solubilizers and colorants.

9. Composition according to any of the preceding claims, wherein said composition comprises a fatty alcohol preferably of the formula R₁OH, R₁ being a aliphatic hydrocarbon group containing 6 to 22 carbon atoms and 0,1,2, or 3 double bonds.

10. Composition according to any of the preceding claims, wherein said composition comprises a fatty alcoholalkoxylate preferably of the formula R₂O(AlkO)ₘH, R₂ being an aliphatic hydrocarbon group containing 6 to 22 carbon atoms, m being an integer from 1 to 30 and AlkO being an alkyleneoxide.

11. Composition according to any of the preceding claims, wherein said composition comprises a fatty alcoholalkoxylate of the formula R₂O(AlkO)ₘH, R₂ being an aliphatic hydrocarbon group containing 8 to 22 carbon atoms, m being an integer from 5 to 20 and AlkO being chosen from ethyleneoxide and propylene oxide.

12. Composition according to any of the preceding claims wherein said composition comprises:
a) 0.01 to 30 % w/w of a thickener,
b) 0.1 to 20 % w/w of a hydrophobic compound having an HLB value of less than 10,
l) 0.5 to 40 % anionic surfactants,
m) 0.25 to 5 % amphoteric surfactants, and/or
n) 0.5 to 40 % nonionic surfactants,
and is further **characterized by** the composition having a weight ratio of components c:d or c:e being in the range of 2:1 to 8:1.

13. Container comprising a cosmetics composition according to any of the preceding claims.

14. Use of a composition according to any of the claims claim 1-14 as a sun cream or lotion, body milk, shampoo, bathing or shower gel, ointment, deodorant, hair care product or moisturizing cream.

## Patentansprüche

1. Kosmetikzusammensetzung, die ein Verdickungsmittel, ein Treibmittel, bei dem es sich um Isopentan handelt, ein Tensid und Wasser umfasst, wobei die Zusammensetzung in einem Behälter unter einem Druck von wenigstens 0,1 bar über Atmosphärendruck und unter 3 bar enthalten ist und wobei die Zusammensetzung in Form eines Gels mit einer Viskosität von 10 000 bis 50 000 mPa·s, gemessen als Brookfield-Viskosität (23 °C, Spindel TE, 5 U/min), vorliegt.

2. Zusammensetzung gemäß Anspruch 1, wobei der Druck nicht größer als 2 bar ist.

3. Zusammensetzung gemäß Anspruch 1 oder 2, wobei der Behälter ein Kunststoffbehälter ist.

4. Zusammensetzung gemäß einem der Ansprüche 1 bis 3, wobei die Zusammensetzung wenigstens zwei Tenside und eine hydrophobe Verbindung mit einem HLB-Wert von weniger als 10 umfasst.

5. Zusammensetzung gemäß einem der vorstehenden Ansprüche, die 0,01 bis 30 Gew.-% Verdickungsmittel, 1 bis 15 Gew.-% Treibmittel, 0,5 bis 50 Gew.-% Tensid und als Rest Wasser sowie weitere übliche Körperpflegebestandteile umfasst.

6. Zusammensetzung gemäß einem der vorstehenden Ansprüche, wobei das Verdickungsmittel aus der Gruppe der Gummen, Poly(meth)acrylate, auf Aerosiltypen beruhenden Polymere, Polysaccharide, hochmolekularen Polyethylenglycolmono- und -diester von Fettsäuren, Polyacrylamide, Polyvinylalkohole, Polyvinylpyrrolidone, Ester von Fettsäuren mit Polyolen, Fettalkoholethoxylate, Alkyloligoglucoside und Zuckerester ausgewählt ist.

7. Zusammensetzung gemäß Anspruch 6, wobei das Verdickungsmittel aus Xanthan, Guarkernmehl, Agaralginaten, Tylosen, Carboxymethylcellulose, Hydroxyethylcellulose ausgewählt ist.

8. Zusammensetzung gemäß einem der vorstehenden Ansprüche, die weiterhin einen oder mehrere Bestandteile umfasst, die aus der Gruppe der pH-Regulatoren, Ölkörper, Emulgatoren, Konservierungsmittel, Duftstoffe, Feuchthaltemittel, UV-Filter, Emollientien, Überfettungsmittel, Aufheller, festigkeitserhöhenden Mittel, Silikonmittel, Fette, Wachse, Lecithine, Phospholipide, Stabilisatoren, antibakteriellen Mittel und anderen bioaktiven Mittel, geruchsabsorbierenden Mittel, Antiperspirantien, Antischuppenmittel, Filmbildner, Quellmittel, Antioxidantien, Insektenabwehrmittel, Hydrotrope, Bräunungsmittel, Tyrosininhibitoren, Lösungsvermittler und Färbemittel ausgewählt sind.

9. Zusammensetzung gemäß einem der vorstehenden Ansprüche, wobei die Zusammensetzung einen Fettalkohol umfasst, vorzugsweise mit der Formel R₁OH, wobei R₁ eine aliphatische Kohlenwasserstoffgruppe ist, die 6 bis 22 Kohlenstoffatome und 0, 1, 2 oder 3 Doppelbindungen enthält.

10. Zusammensetzung gemäß einem der vorstehenden Ansprüche, wobei die Zusammensetzung ein Fettalkoholalkoxylat umfasst, vorzugsweise mit der Formel R₂O(AlkO)ₘH, wobei R₂ eine aliphatische Kohlenwasserstoffgruppe ist, die 6 bis 22 Kohlenstoffatome enthält, m eine ganze Zahl von 1 bis 30 ist und AlkO ein Alkylenoxid ist.

11. Zusammensetzung gemäß einem der vorstehenden Ansprüche, wobei die Zusammensetzung ein Fettalkoholalkoxylat der Formel R₂O(AlkO)ₘH umfasst, wobei R₂ eine aliphatische Kohlenwasserstoffgruppe ist, die 8 bis 22 Kohlenstoffatome enthält, m eine ganze Zahl von 5 bis 20 ist und AlkO aus. Ethylenoxid und Propylenoxid ausgewählt ist.

12. Zusammensetzung gemäß einem der vorstehenden Ansprüche, wobei die Zusammensetzung Folgendes umfasst:
a) 0,01 bis 30 Gew.-% eines Verdickungsmittels;
b) 0,1 bis 20 Gew.-% einer hydrophoben Verbindung mit einem HLB-Wert von weniger als 10;
l) 0,5 bis 40% anionische Tenside;
m) 0,25 bis 5% amphotere Tenside; und/oder
n) 0,5 bis 40% nichtionische Tenside;
und weiterhin **dadurch gekennzeichnet ist, dass** die Zusammensetzung ein Gewichtsverhältnis von Komponenten c:d oder c:e aufweist, das im Bereich von 2:1 bis 8:1 liegt.

13. Behälter, der eine Kosmetikzusammensetzung gemäß einem der vorstehenden Ansprüche umfasst.

14. Verwendung einer Zusammensetzung gemäß einem der Ansprüche 1 bis 14 als Sonnencreme oder -lotion, Körpermilch, Shampoo, Bade- oder Duschgel, Salbe, Deodorant, Haarpflegeprodukt oder Feuchtigkeitscreme.

## Revendications

1. Composition cosmétique comprenant un agent épaississant, un agent propulseur qui consiste en l'iso-pentane, un agent tensioactif et de l'eau, ladite composition étant contenue dans un conteneur sous une pression d'au moins 0,1 bar sde plus que la pression atmosphérique et inférieure à 3 bars, et ladite composition étant sous la forme d'un gel dont la viscosité est comprise entre 10 000 et 50 000 mPas, cette viscosité étant exprimée en viscosité Brookfield (23°C, broche TE, 5 Rpm).

2. Composition selon la revendication 1, dans laquelle la pression est inférieure à 2 bars.

3. Composition selon la revendication 1 or 2, dans laquelle le conteneur est un conteneur en matière plastique.

4. Composition selon l'une quelconque des revendications 1-3, dans laquelle ladite composition comprend au moins deux agents tensioactifs et un composé hydrophobe présentant une valeur HLB inférieure à 10.

5. Composition selon l'une quelconque des revendications précédentes, comprenant de 0,01 à 30 % en poids d'agent épaississant, de 1 à 15% en poids d'agent propulseur, de 0,5 à 50 % en poids d'agent tensioactif et le reste consistant en eau et autres ingrédients usuels pour les soins corporels.

6. Composition selon l'une quelconque des revendications précédentes, dans laquelle l'agent épaississant est choisi dans le groupe comprenant les gommes, les poly(méth)acrylates, les polymères de types aérosil, les polysaccharides, les mono- et di-esters d'acides gras de polyéthylèneglycol de poids moléculaire élevé, les polyacrylamides, les alcools polyvinyliques, les polyvynylpyrrolidones, les esters d'acides gras avec des polyols, les éthoxylates d'alcools gras, les alkyloligoglucosides et les esters de sucres.

7. Composition selon la revendication 6, dans laquelle l'agent épaississant est choisi dans le groupe comprenant la gomme de xanthane, guar-guar, alginates d'agar-agar, tyloses, carboxyméthylcellulose, hydroxyéthylcellulose.

8. Composition selon l'une quelconque des revendications précédentes comprenant en outre un ou plusieurs ingrédients choisis dans le groupe comprenant les agents de régulation du pH, des corps huileux, des agents émulsifiants, des conservateurs, des parfums, des agents hydratants, des filtres aux UV, des émollients, des agents surgraissants, des agents azurants, des agents d'amélioration de la résistance, des agents à base de silice, des graisses, des cires, des lécithines, des phospholipides, des agents stabilisants, des agents anti-bactériens et autres agents bioactifs, des agents d'absorption des odeurs, des antitranspirants, des agents antipelliculaires, des agents filmogènes, des agents de gonflement, des antioxydants, des insecticides, des hydrotropes, des agents tinctoriaux, des inhibiteurs de tyrosine, des agents de solubilisation et des colorants.

9. Composition selon l'une quelconque des revendications précédentes, dans laquelle ladite composition comprend un alcool gras, de préférence de formule R₁OH, R₁ étant un groupe hydrocarboné aliphatique contenant 6 à 22 atomes de carbone et 0,1,2, or 3 double liaisons.

10. Composition selon l'une quelconque des revendications précédentes, dans laquelle ladite composition comprend un alkoxylate d'alcool gras, de préférence de formule R₂O(AlkO)ₘH, R₂ étant un groupe hydrocarboné aliphatique contenant 6 à 22 atomes de carbone, m étant un nombre entier compris entre 1 et 30 et AlkO étant un oxyde d'alkylène.

11. Composition selon l'une quelconque des revendications précédentes, dans laquelle ladite composition comprend un alkoxylate d'alcool gras de formule R₂O(AlkO)ₘH, R₂ étant un groupe hydrocarboné aliphatique contenant 8 à 22 atomes de carbone, m étant un nombre entier compris entre 5 et 20 et AlkO étant un oxyde d'éthylène ou de propylène.

12. Composition selon l'une quelconque des revendications précédentes dans laquelle ladite composition comprend:
a) 0,01 à 30 % en poids d'un agent épaississant,
b) 0.1 to 20 % en poids d'un composé hydrophobe ayant une valeur HLB inférieure à 10,
l) 0.5 to 40 % d'un agent tensioactif anionique,
m) 0.25 to 5 % d'un agent tensioactif amphotère, et/ou
n) 0.5 to 40% d'un agent tensioactif non ionique
et ladite composition étant en outre **caractérisée en ce que** le rapport pondéral des composants c/d ou c/e est compris entre 2/1 et 8/1.

13. Conteneur comprenant une composition cosmétique selon l'une quelconque des revendications précédentes.

14. Utilisation d'une composition selon l'une quelconque des revendications 1-14 en tant que crèmes ou lotions solaires, laits corporels, shampooings, gels de bain ou de douche, onguents , déodorants, produits capillaires ou crèmes hydratantes.
